(19) Europäisches Patentamt
European Patent Office
Office européen des brevets

(11) Publication number: **0 173 356**
**B1**

(12) # EUROPEAN PATENT SPECIFICATION

(45) Date of publication of patent specification: **19.09.90**

(51) Int. Cl.$^5$: **C 07 H 21/00**

(21) Application number: **85200673.3**

(22) Date of filing: **27.02.81**

(60) Publication number of the earlier application in accordance with Art. 76 EPC: **0 035 719**

(54) **Process for preparing modified inorganic polymers.**

(30) Priority: **29.02.80 US 126025**

(43) Date of publication of application:
**05.03.86 Bulletin 86/10**

(45) Publication of the grant of the patent:
**19.09.90 Bulletin 90/38**

(84) Designated Contracting States:
**AT BE CH DE FR GB IT LI NL**

(56) References cited:
**EP-A-0 040 099**

**TETRAHEDRON LETTERS, no. 16, 1972, pages 1527-1530, Pergamon Press, Oxford, GB; H. KÖSTER: "Polymer support oligonucleotide synthesis. Use of inorganic carriers"**

**CHEMICAL REVIEWS, vol. 77, no. 2, April 1977, pages 183-217, Columbus, Ohio, US; V. AMARNATH et al.: "Chemical synthesis of oligonucleotides"**

(73) Proprietor: **UNIVERSITY PATENTS, INC.**
**537 Newtown Avenue**
**Norwalk Connecticut 06851 (US)**

(72) Inventor: **Caruthers, Marvin H, Dr.**
**1450 Kendall Drive**
**Boulder Colorado (US)**

(74) Representative: **Allam, Peter Clerk et al**
**LLOYD WISE, TREGEAR & CO. Norman House**
**105-109 Strand**
**London WC2R 0AE (GB)**

(56) References cited:
**TETRAHEDRON LETTERS, vol. 21, 1980, pages 4159-4162, Pergamon Press Ltd., Oxford, GB; K.K. OGILVIE et al.: "Silica gel as solid support in the synthesis of oligoribonucleotides"**

**TETRAHEDRON LETTERS, vol. 21, 1980, pages 719-722, Pergamon Press Ltd., Oxford, GB; M.D. MATTEUCCI et al.: "The synthesis of oligodeoxypyrimidines on a polymer support"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 173 356 B1**

Courier Press, Leamington Spa, England.

**Description**

The present invention relates to a process for preparing modified inorganic polymers which are useful as a support structure in methods of producing sequence defined oligonucleotides.

Numerous attempts have been made to develop a successful methodology for synthesizing sequence defined oligonucleotides. However, the stepwise synthesis of polynucleotides, and specifically oligonucleotides still remains a difficult and time consuming task, often with low yields. One prior art technique has included the use of organic polymers as supports during polynucleotide synthesis. Classically the major problems with polymer supported synthesis strategies has been inherent in the nature of the polymer support. Various prior art polymers used in such syntheses have proven inadequate for reasons such as: (1) slow diffusion rates of activated nucleotides into the support; (2) excessive swelling of various macroporous, low crosslinked support polymers; and (3) irreversible absorption of reagents onto the polymer. See for example, V. Amarnath and A. D. Broom, *Chemical Reviews 77*, 183—217 (1977).

Modified inorganic polymers are known in the prior art, primarily for use as absorption materials, for example, in liquid chromatography. The attachment of nucleosidephosphates to silica gel using a trityl linking group is described in the prior art (H. Koster, *Tetrahedron* Letters, 1527—1530, 1972) but the method is apparently applicable only to pyrimidine nucleosides. The cleavage of the nucleoside from the silica support can only be accomplished with acid to which the purine nucleosides are sensitive.

Recently, Matteucci and Caruthers have published (see Tetrahedon Letters, Vol. 21, pages 719—722) a procedure for preparing a thymidine-modified inorganic polymer useful as a support for the synthesis of sequence defined oligonucleotides. The procedure described involves a number of steps. The initial step comprises refluxing 3-aminopropyltriethoxysilane with macroporous hplc silica gel in dry toluene for 3 hours, whereafter succinic anhydride in water is reacted with the amino silica gel formed in the first step while the pH is maintained at pH 4—6. Next, excess silicic acid functionality is eliminated by treatment with $(CH_3)_3SiCl$ in anhydrous pyridine for 12 hours. Thymidine is then linked to the carboxylic acid support through the 3′-OH via an ester bond. To accomplish this linkage, 5′-O-dimethoxytritylthymidine is condensed with the derivatized silica gel in anhydrous pyridine using dicyclohexylcarbodiimide as condensing agent. After 40 hours, residual acid groups are converted to an inert amide and the dimethoxytrityl group is removed. The modified inorganic polymer thus obtained can be represented by the formula:

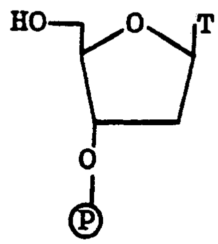

wherein T represents the attached nucleoside base and Ⓟ the silica gel support. The yield of thymidine attached to the support is reported to be 40 µmole/g.

The present invention provides a new process for producing such inorganic polymers.

In general, the present invention relates to a process for preparing a modified inorganic polymer represented by one of the formulae:

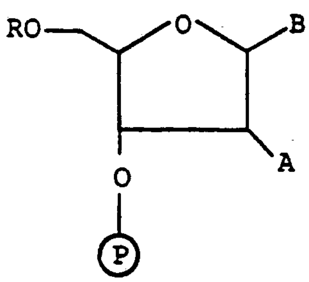 and 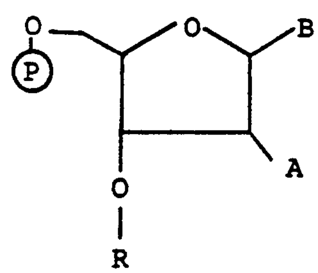

wherein B is a nucleoside or deoxynucleoside base; Ⓟ represents an inorganic polymeric support linked to the 3′- or 5′-O- of the nucleoside through an amide bond; R is H or a blocking group; and A is H or OR, which process comprises the steps of:

(1) converting an inorganic polymeric support material to a matrix containing aminoalkyl groups;

(2) blocking unreacted groups in said support material;

(3) providing a nucleoside or deoxy nucleoside which has been modified at one of the 3′- or 5′-positions to contain the half-ester of a dicarboxylic acid and which has been blocked at the other of the 3′- or 5′-OH positions;

(4) coupling the derivatized support obtained in step (2) to said modified nucleoside or deoxynucleoside provided in step (3) by forming an amide link between the free carboxy group of the half-ester group of said modified nucleoside or deoxynucleoside and the free amino group of said derivatized support;

(5) blocking unreacted hydroxy groups on the support; and optionally

(6) converting the blocked 3'- or 5'-OH group on the nucleoside or deoxynucleoside to a free hydroxyl group.

Compared to the prior art process of the Tetrahedron Letters article, *supra*, the process of the present invention has two distinct advantages. Firstly, the present process is preferred since it leads to more control of the amount of the nucleoside loaded onto the inorganic polymer support. Additionally, the present process can lead to more nucleoside being joined to the polymer support (e.g. approximately 100—120 µmole/g of silica gel compared to 10—40 µmole/g of silica gel by the prior process).

Of course, the nucleoside moiety of the present modified inorganic polymers can include more than one nucleoside and may include a number of nucleosides condensed as oligonucleotides with the oligonucleotide being attached to the inorganic polymer support through the single chemical linkage i.e. amide linkage.

The thus modified inorganic polymer supports are useful in the stepwise addition of nucleosides or oligonucleotides to the original nucleoside moiety of the support by a series of process steps as described in detail in our co-pending European Patent Application No. 81101445.5; EP—A—0035719 from which the present application is divided. Subsequently, the polynucleotides so produced are released from the polymer support.

A wide variety of inorganic polymers can be employed in the present invention and these include, for example, silica, porous glass, aluminosilicates, borosilicates, metal oxides such as alumina and nickel oxide, and various clays. The polymer should be substantially insoluble in the reaction solvents employed and relatively chemically inert to the reagents to be employed during subsequent processing, except of course for the chemical reactivity required to form the hereinbefore-described chemical bond with the initial nucleoside through which the eventual polynucleoside is attached to the support.

While the invention can be implemented with a variety of inorganic polymers, it will be described herein in more detail utilizing silica gel as the polymer support. A particularly preferred silica gel is macroporous silica which is used in high performance liquid chromatography (hplc). In addition, the invention will be described using deoxynucleotides but it should be understood that ribonucleotides can be substituted therefor to obtain similar results.

As employed herein, the terms nucleoside, nucleotide and oligonucleotide are intended to include the deoxy counterparts which differ only in the absence of a hydroxy group in the 2' position. Thus, these terms include structures wherein the 2' position substituent is H or OH (as shown hereinafter by substituent A in formulae I, II and III).

The nucleoside is linked to the silica gel support through an amide bond which therefore is readily hydrolyzable with a weak base such as ammonium hydroxide. This linkage is accomplished by pre-forming a nucleoside which has been modified at one of the 3'- or 5'-OH positions to contain the half-ester of a dicarboxylic acid, the other of the 3'- or 5'-OH positions being blocked, followed by coupling of the modified nucleoside to the support by forming an amide link between the free carboxy group of the half-ester and the free amino group of the support. Thus, the process involves the following steps:

(1) conversion of silica gel to matrix containing aminoalkyl groups;

(2) block unreacted silanol OH groups with suitable blocking agents, e.g. trialkylhalosilanes such as trimethylchlorosilane or the bromo analog;

(3) join the free amino group of the derivatized silica gel to the free carboxy group of a selected nucleoside which has been modified to contain the half ester of a dicarboxylic acid to form an amide bond; and

(4) blocking unreactive amino groups on the silica gel support, e.g. unreacted amino groups of the amino-derivatized silica gel are preferably blocked by acylation with monocarboxylic acids such as acetic, benzoic or isobutyric acids, normally employing the acid anhydrides under acylating conditions.

Preferably, the nucleoside is linked to the silica gel through the 3'-OH group rather than the 5'-OH leaving the 5'-OH available for subsequent linkage to another nucleoside. Thus, linkage of the added nucleoside occurs at the 3'-OH group and the 5'-OH remains available for linkage to a further added nucleoside.

Accordingly, to accomplish the desired linkage at the 3'-OH and 5'-OH respectively, the initial nucleoside is linked through the 3'-OH to the silica gel by the coupling reaction previously defined herein. This is accomplished by blocking the 5'-OH e.g. by use of trityl groups, such as the dimethoxytrityl group, which are preferred since they are readily removed after the initial 3'-OH coupling reaction occurs.

When the initial nucleoside includes nitro groups, e.g. guanosine, adenosine, cytidine, deoxyguanosine, deoxyadenosine and deoxycytidine, it is preferred to block these groups using known acylating techniques, e.g. with acetic acid, benzoic acid, isobutyric acid and like acids and such blocking group can be removed when convenient, usually after the final oligonucleotide is obtained.

The aminoalkyl groups are incorporated on the silica gel by reaction of aminoalkyl-trialkoxysilane which is conveniently accomplished by refluxing in a solvent, e.g. toluene, for several hours. Suitable

reagents include aminopropyltriethoxysilane, 4-aminobutyltrimethoxysilane, 4-aminobutyltriethoxysilane and 2-aminoethyltriethoxysilane.

The preferred method for the present invention is to introduce amino functionality on the silica by reaction with an aminoalkyl silane derivative, e.g. by reaction of a trialkoxy 3-aminopropylsilane such as triethoxy 3-amino-propylsilane with the silica support to form the covalent linkage:

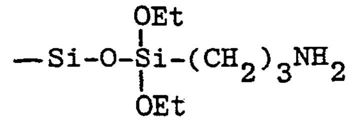

The dicarboxylic acid employed in forming the linkage of nucleoside to the silica gel can be any of a variety such as succinic, glutaric, adipic, phthalic, maleic and similar such dicarboxylic acids of the aliphatic or aromatic type containing preferably up to about 10 carbon atoms. Esterification with the dicarboxylic acid is best accomplished by using the acid anhydride to assure monoesterification.

The product produced, i.e. the nucleoside-modified silica gel, can be represented by the following formula:

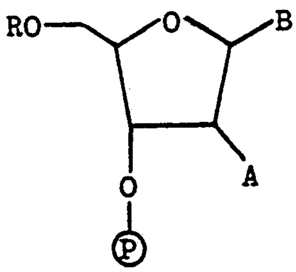

wherein B is the nucleoside or deoxynucleoside base; and Ⓟ represents the silica support and the amide covalent linking group, which is preferably represented by the formula:

$$\text{(silica gel)}\text{———}(CH_2)_nNHCO\ Z\ CO\text{—}$$

in which n is an integer from 1—5 and Z is divalent hydrocarbyl radical including alkyl, alkenyl, cycloalkyl, aryl and aralkyl of up to about 10 carbon atoms; A is H or OR; and R is H or a blocking group, e.g. trityl, methoxytrityl, dimethoxytrityl, dialkylphosphite, pivalylisobutyloxycarbonyl, t-butyldimethylsilyl, and similar such blocking groups.

The silica gel which is used as starting material in the production of the modified silica gels of the present invention is not critical. Silica gel particles in the range of from about 5 µm to about 1000 µm are useful, with particles in the range of about 10 µm to about 50 µm being preferred. In a similar manner pore size is not critical. Pore sizes in the range of about 50 Å to about 2000 Å are preferred.

The blocked 3'- or 5'-hydroxy groups of the modified polymers prepared by the process of the present invention may be converted to free hydroxyl groups so that the free hydroxyl group becomes available for condensation with a further selected nucleoside.

The modified silica gels of the present invention: (1) allow relatively rapid diffusion of activated nucleotides, and other reagents into the support; (2) avoid swelling; and (3) resist adsorption of reagents. Additionally, these modified silica gels obtainable by the present invention are (1) insoluble in most solvents; (2) in use as support matrices, allow solvents and unwanted reaction products to be easily washed from the matrix, while maintaining the desired reaction products in place and capable of continuous processing; and (3) allow the supported material to react relatively rapidly and in high yield, for example, in cylindrical condensation.

As used herein the symbols for nucleotides and polynucleotides are according to the IUPAC-IUB Commission of Biochemical Nomenclature Recommendations [(1970 *Biochemistry 9*, 4022].

The following examples further illustrate the invention.

## Comparative Example

Polymer supports functionalized with carboxylic acid groups are prepared from silica gel by the method described in the Tetrahedron Letters paper aforesaid.

A separation group silica gel supplied by Vydak as TP silica, having 20 µm particle size, 300 Å pore size is used as the starting material. The silica gel was placed in a desiccator over a saturated LiCl solution for 24 hours. The initial step in the process is silylation by refluxing 3-aminopropyltriethoxysilane (2.3 g, 0.01 M) with the silica gel (2.6 g) in dry toluene. After silylation is substantially complete, in this case after about twelve hours of refluxing, the reaction mixture is cooled and the toluene solution removed. The thus silylated silica gel is then washed serially with toluene, ethanol and then with ether and air dried. Succinic anhydride (2.5 g, 0.025 M) in water is next reacted with the silane modified silica gel to provide carboxylic

acid functionality to the terminal portion of the covalently bonded silane side chains. During this latter reaction, the pH is maintained between 4 and 6 by addition of a base, such as 2 N sodium hydroxide. After this latter reaction, which proceeded for about 6 hours, the modified silica gel containing carboxylic acid functional groups on its side chains is washed with water, then with methanol and ether, and then finally dried in vacuum at room temperature. The modified silica gel is then treated with trimethylsilylchloride [$(CH_3)_3SiCl$, 1.09 g, 0.01 M] in anhydrous pyridine by refluxing for about 12 hours. The resulting modified silica gel is then washed with 5% trichloroacetic acid in water, then with water, and then with ethanol and ether. After drying in vacuum, the yield of carboxylic acid functionality on the modified silica gel is about 250 μmole/g.

5'-O-dimethoxytrityldeoxythymidine (1.17 g, 0.002 M) and the modified silica gel described in A (4 g, 0.001 mole carboxylic acid functional group) are reacted for about 40 hours in anhydrous pyridine using dicyclohexycarbodiimide (2.06 g, 0.01 M) as condensing agent. The unreacted residual carboxylic acid groups in the modified silica are blocked, by the addition of p-nitrophenol (1.4 g, 0.01 M) followed by the addition of 10% piperidine in pyridine (25 minutes). The reaction product is then washed serially with tetrahydrofuran, methanol and finally with ethyl ether. Then, as a precaution to assure complete blockage of unreacted carboxylic acid, the composition is first treated with dicyclohexylcarbodiimide and p-nitrophenol and then piperidine in pyridine for a second time. After removal of the dimethoxytrityl group using 0.1 N p-toluenesulfonic acid in acetonitrile, the yield of thymidine attached to the support is found by spectrophotometry to be about 40 μmole/g.

Example

A. Silica gel (Vydac A, 25 gms) was placed in a desiccator over a saturated LiCl solution for 24 hours. The silica gel was transferred to a 500 ml round bottom flask, toluene (250 ml) and aminopropyltriethoxysilane (13 ml) were added, the flask was tightly sealed, and the suspension was gently shaken for 12 hours at room temperature. The flask containing the suspended silica gel was next refluxed for 18 hours. Before starting the reflux, add one boiling chip to the solution. Following the reflux step, the silica gel suspension was transferred to a centrifuge bottle and the silica gel pelleted by a low speed spin. The supernatant was decanted and the silica gel was washed with toluene (3×, 80 ml ea), methanol (3×, 80 ml ea) and methanol:$H_2O$, 1:1 (2×, 80 ml ea). The silica gel was next suspended in 80 ml 50% aqueous methanol and shaken overnight at room temperature. Once again the silica gel suspension was isolated by transfer to a centrifuge bottle followed by a low speed spin. The silica gel was washed with methanol (2×, 80 ml ea) and ethyl ether (3×, 80 ml ea). Finally, the silica gel was air dried for 6 hours and then dried *in vacuo*.

The silica gel was placed in a round bottom flask. A solution of dry pyridine (50 ml) and trimethylsilyl chloride was added and the suspension shaken at room temperature overnight. The silica was isolated by low speed centrifugation. The silica was then washed with methanol (5×, 80 ml) and ethyl ether (3×, 80 ml). The silica gel was air dried for 6 hours and then dried *in vacuo*.

B. The 5'-O-dimethoxytrityl and N-protected deoxynucleoside (2.5 mole) was dissolved in a solution of dry pyridine (5 ml) and N,N-dimethylaminopyridine (0.3 g). Succinic anhydride (2.0 mmole, 0.2 g) was added and the solution stirred at room temperature for 12 hours. Thin layer chromatography (tlc) in acetonitrile:water (9:1, v/v) can be used to monitor the reaction. Unreacted nucleoside will have an $R_f$ of approximately 0.8 whereas the product will be a smear from $R_f$ 0.3 to $R_f$ 0.5. After completion of the reaction, solvent is removed in a rotary evaporator and the dry gum is redissolved in toluene (10 ml). Toluene is removed using a rotary evaporator and the toluene co-evaporation procedure is repeated. The dry gum free of pyridine and N,N-dimethylaminopyridine is dissolved in methylenechloride (30 ml). This solution is transferred to an extraction funnel and 10% ice-cold citric acid is added. After vigorous shaking and extraction, the organic phase is washed twice with water (15 ml ea) and then dried over sodium sulfate. Approximately 0.3 ml pyridine is added to the methylene chloride solution in order to minimize detritylation while drying over sodium sulfate. The methylene chloride solution is concentrated to 10 ml and the succinylated nucleoside isolated by precipitation into hexane:ether (1:1, v/v; 250 ml). The precipitate is collected by centrifugation and dried *in vacuo*.

To obtain the nitrophenyl esters, succinylated nucleoside (1 mmole) was dissolved in dry dioxane (3 ml) containing pyridine (0.3 ml). DCC (10 mmole, 0.22 g) and p-nitrophenol (0.14 g, 1 mmole) were added and the solution shaken for 2 hours. Dicyclohexyl urea was removed by centrifugation. Analysis by tlc in acetonitrile:$H_2O$ (9:1, v/v) indicates the product with an $R_f$ of 0.8. This supernatant free of dicyclohexylurea is used directly for coupling to silica gel.

Silica gel prepared as outlined in A of this example (5 g if 50 μmole nucleoside/g desired; 2.5 g if 100 μmole nucleoside/g desired) was suspended in dry DMF. The p-nitrophenylsuccinylated nucleoside derivative (supernatant prepared herein) was added to the silica gel and the resulting suspension was shaken for two hours. An aliquot of silica gel (approx. 1 mg) was then removed for analysis. After washing the aliquot with DMF (2×), methanol (3×) and ethyl ether (2×), 0.1 M toluene-sulfonic acid in acetonitrile (1 ml) was added to the aliquot and the trityl released from silica as a red-orange color was observed. This analysis can be completed quantitatively if desired. If this analysis appears satisfactory (i.e. a positive trityl test), the bulk of the silica gel was washed with DMF (3×, 10 ml ea), dioxane (3×, 10 ml ea), methanol (5×, 10 ml ea), and ethyl ether (3×, 10 ml ea). Unreacted n-propylamino silyl groups were then blocked with a solution of acetic anhydride (0.7 ml) and dry pyridine (5 ml). The silica gel was isolated by centrifugation, decanting and repeated washing with methanol (4×, 10 ml ea) and ethyl ether (2×, 10 ml ea).

The assay for completeness of the capping or blocking of n-propylamino groups is as follows.

Take an aliquot (1 mg) of: (1) Underivatized Vydac-A, (2) Vydac derivatized with the aminopropyltriethoxysilane, (3) Vydac that has had nucleoside attached and subsequently blocked with acetic anhydride. Each sample was then treated with 250 μl of saturated sodium borate containing 0.2 mg/ml picryl sulfate. Vortex and centrifuge the reactant products. The underivatized Vydac should remain white. The aminopropylsilyl Vydac should appear bright orange-red. The capped Vydac will be pale yellow-orange. This is probably due to interaction of picryl sulfate with ring nitrogens on nucleosides.

With some preparations, a contaminant of succinylated n-propylamino groups will result from the presence of succinic acid. This succinic acid may be present because all the succinic anhydride was not consumed during the succinylation or alternatively was not removed as succinic acid during the aqueous extraction with citric acid. If succinylated n-propylamino groups are present, they can be blocked in the following manner. The protected silica gel containing succinylated nucleoside (either 5 g or 2.5 g) was suspended in a solution of dry pyridine (5 ml) containing DCC (0.28 g) and p-nitrophenol (0.16 g) and shaken overnight at room temperature. Morpholine (0.2 ml) was then added and the suspension shaken for 10 minutes. Silica gel was isolated after centrifugation, decantation of the supernatant, and washing the silica gel with methanol (4×, 10 ml ea), THF (3×, 10 ml ea) and ethyl ether (3×, 10 ml ea). After air drying, the silica gel was dried *in vacuo*.

A quantitative assay for the trityl cation and therefore the loading of nucleoside on the silica gel is as follows:

1. Weigh accurately approximately 1 mg of dry silica gel.
2. Add 1 ml of 0.1 M toluenesulfonic acid in acetonitrile.
3. Measure the absorbance at 498 nm. If the absorbance approaches 2.0, dilute and re-read. The loading can be calculated as follows:

$$\text{loading in } \mu\text{moles/g} = \frac{(\text{Abs}^{498})\ (\text{dilution factor})}{\text{wt silica gel in mg}} \times 14.3$$

If 5 gm silica gel was used, the loading should be approximately 40 μmole/g. If 2.5 gm silica gel was used, the loading will be approximately 100 μmole/g.

**Claims**

1. A process for preparing a modified inorganic polymer represented by one of the formulae:

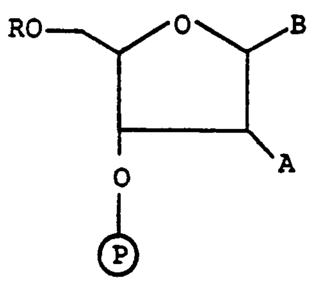

and

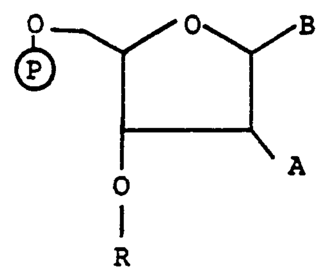

wherein B is a nucleoside or deoxynucleoside base; Ⓟ represents an inorganic polymeric support linked to the 3'- or 5'-O- of the nucleoside through an amide bond; R is H or a blocking group; and A is H or OR, which process comprises the steps of:

(1) converting an inorganic polymeric support material to a matrix containing aminoalkyl groups;

(2) blocking unreacted groups in said support material;

(3) providing a nucleoside or deoxy nucleoside which has been modified at one of the 3'- or 5'-positions to contain the half-ester of a dicarboxylic acid and which has been blocked at the other of the 3'- or 5'-OH positions;

(4) coupling the derivatized support obtained in step (2) to said modified nucleoside or deoxynucleoside provided in step (3) by forming an amide link between the free carboxy group of the half-ester group of said modified nucleoside or deoxynucleoside and the free amino group of said derivatized support;

(5) blocking unreacted amino groups on the support; and optionally

(6) converting the blocked 3'- or 5'-OH group on the nucleoside or deoxynucleoside to a free hydroxy group.

2. A process according to Claim 1, wherein said inorganic polymeric support is selected from silica, porous glass, aluminosilicates and borosilicates.

3. A process according to Claim 2, wherein said inorganic polymeric support is a macroporous silica gel.

4. A process according to any preceding claim, wherein said nucleoside or deoxynucleoside used in step (3) is blocked at the 5'-OH.

5. A process according to any preceding claim, wherein said nucleoside or deoxynucleoside used in step (3) is blocked in either the 3'- or the 5'-OH by a trityl group.

6. A process according to any preceding claim, wherein unreacted silanol OH groups are blocked in step (2) by means of a trialkylhalosilane.

7. A process according to any preceding claim, wherein the amide covalent group linking said inorganic polymer to said 3'- or 5'-O of the nucleoside is represented by the formula:

$$—(CH_2)_nNHCOZCO—$$

wherein n is an integer from 1—5 and Z is a divalent hydrocarbyl radical.

8. A process according to Claim 7, wherein said amide covalent group is the group:

$$—(CH_2)_3NHCO(CH_2)_2CO—.$$

9. A process according to any preceding claim, wherein said nucleoside or deoxynucleoside which is provided in step (3) has the formula:

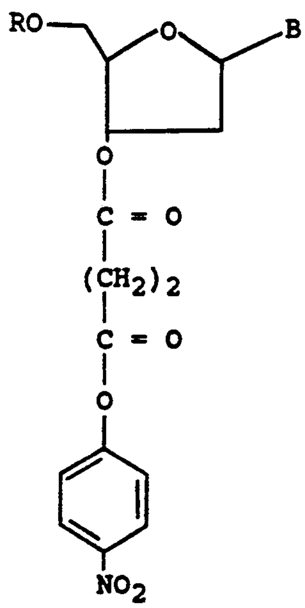

wherein R is a blocking group and B is as defined in Claim 1.

**Patentansprüche**

1. Verfahren zum Herstellen eines modifizierten anorganischen Polymers nach der Darstellung einer der Formeln:

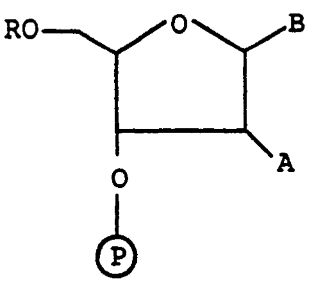

und

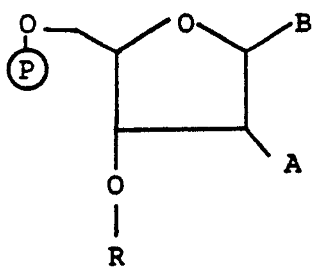

wobei B eine Nukleosid- oder Desoxynukleosidbase ist, Ⓟ einen über eine Amidbindung mit dem 3'- oder 5'-O- des Nukleosids verketteten anorganischen polymeren Träger darstellt, R ein H oder eine Blockinggruppe und A ein H oder OR ist, gekennzeichnet durch die Verfahrensschritte des:

(1) Umwandelns eines anorganischen polymeren Trägermaterials zu einer Aminoalkylgruppen enthalten Matrix,

(2) Blockierens nichtumgesetzter Gruppen im Trägermaterial,

(3) Bereitstellens eines Nukleosids oder Deosoxynukleosids, das an einer der 3'- oder 5'-Stellungen

modifiziert wurde, um den Halbester einer Dikarbonsäure zu enthalten, und das an der anderen der 3'- oder 5'-Stellungen blockiert wurde,

(4) Verknüpfens des aus dem Verfahrensschritt (2) abgeleiteten Trägers mit dem durch den Verfahrensschritt (3) bereitgestellten modifizierten Nukleosid oder Desoxynukleosid, indem eine Amidverkettung zwischen der freien Karboxylgruppe der Halbestergruppe des modifizierten Nukleosids oder Desoxynukleosids und der freien Aminogruppe des abgeleiteten Trägers gebildet wird,

(5) Blockierens nichtumgesetzter Aminogruppen auf dem Träger und nach Wahl,

(6) Umwandelns der blockierten 3'- oder 5'-OH-Gruppe auf dem Nukleosid oder Desoxynukleosid zu einer freien Hydroxylgruppe.

2. Verfahren nach Anspruch 1, dadurch gekennzeichnet, daß als anorganischer polymerer Träger Silika, poröses Glas, Alumosilikate und Borosilikate verwendet werden.

3. Verfahren nach Anspruch 2, dadurch gekennzeichnet, daß der anorganische polymere Träger ein makroporöser Kieselsäuregel ist.

4. Verfahren nach einem der vorhergehenden Ansprüche 1 bis 3, dadurch gekennzeichnet, daß das im Verfahrensschritt (3) verwendete Nukleosid oder Desoxynukleosid an der 5'-OH blockiert ist.

5. Verfahren nach einem der vorhergehenden Ansprüche 1 bis 4, dadurch gekennzeichnet, daß das im Verfahrensschritt (3) verwendete Nukleosid oder Desoxynukleosid durch eine Tritylgruppe entweder bei 3'- oder 5'-OH blockiert ist.

6. Verfahren nach einem der vorhergehenden Ansprüche 1 bis 5, dadurch gekennzeichnet, daß die nichtumgesetzten Silanol-OH-Gruppen im Verfahrensschritt (2) mittels eines Trialkylhalosilans blockiert sind.

7. Verfahren nach einem der vorhergehenden Ansprüche 1 bis 6, dadurch gekennzeichnet, daß die das anorganische Polymer mit dem 3'- oder 5'-O des Nukleosids verkettende amidkovalente Gruppe dargestellt wird durch die Formel:

$$—(CH_2)_nNHCOZCO—,$$

wobei n eine ganze Zahl von 1 bis 5 und Z ein zweiwertiges Hydrokarboxylradikal ist.

8. Verfahren nach Anspruch 7, dadurch gekennzeichnet, daß die amidkovalente Gruppe die Gruppe:

$$—(CH_2)_3NHCO(CH_2)_2CO—$$

ist.

9. Verfahren nach einem der vorhergehenden Ansprüche 1 bis 8, dadurch gekennzeichnet, daß das im Verfahrensschritt (3) bereitgestellte Nukleosid oder Desoxynukleosid die Formel

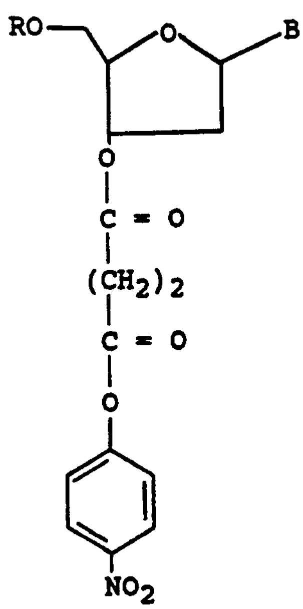

hat, worin R eine Blockinggruppe und B nach Anspruch 1 definiert ist.

**Revendications**

1. Procédé pour préparer un polymère minéral ("inorganique") modifié, représenté par l'une des formules

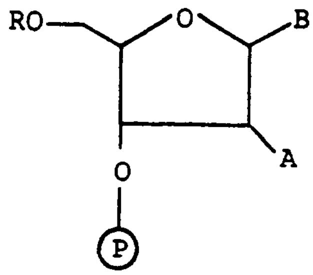 et 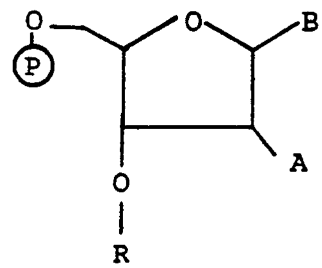

(dans lesquelles B représente une base de nucléoside ou de désoxynucléoside; Ⓟ représente un support polymère minéral relié à la position 3'- ou 5'-O- du nucléoside par une liaison par chaînon amide; R représente H ou un groupe de blocage; et A représente H ou OR), ce procédé comprenant les étapes consistant à:

(1) convertir une matière de support polymère minéral en une matrice contenant des groupes aminoalkyles;

(2) bloquer des groupes n'ayant pas réagi dans ladite matière de support;

(3) fournir un nucléoside, ou un désoxynucléoside, qui a été modifié sur l'une des positions 3' ou 5' de façon qu'il contienne l'hémi-ester d'un acide dicarboxylique, et qui a été bloqué sur l'autre des positions 3'- ou 5'-OH;

(4) coupler le support, obtenu sous forme de dérivé à l'étape (2), audit nucléoside ou désoxynucléoside modifié, fourni à l'étape (3), en formant une liaison amide entre le groupe carboxy libre du groupe hémi-ester dudit nucléoside ou désoxynucléoside modifié et le groupe amino libre dudit dérivé de support;

(5) bloquer sur le support les groupes amino n'ayant pas réagi; et éventuellement

(6) convertir le groupe 3'-OH ou 5'-OH bloqué du nucléoside ou du désoxynucléoside en un groupe hydroxy libre.

2. Procédé selon la revendication 1, dans lequel ledit support polymère minéral est choisi parmi de la silice, du verre poreux, des aluminosilicates et des borosilicates.

3. Procédé selon la revendication 2, dans lequel ledit support polymère minéral est un gel de silice macroporeuse.

4. Procédé selon l'une quelconque des revendications précédentes, dans lequel ledit nucléoside ou désoxynucléoside utilisé à l'étape (3) est bloqué sur le groupe OH en position 5'.

5. Procédé selon l'une quelconque des revendications précédentes, dans lequel ledit nucléoside ou désoxynucléoside utilisé à l'étape (3) est bloqué par un groupe trityle sur le groupe —OH en position 3' ou en position 5'.

6. Procédé selon l'une quelconque des revendications précédentes, dans lequel les groupes OH de silanol n'ayant pas réagi sont bloqués à l'étape (2) à l'aide d'un trialkylhalogénosilane.

7. Procédé selon l'une quelconque des revendications précédentes, dans lequel le groupe amide covalent reliant ledit polymère minéral à ladite position 3'-O ou 5'-O du nucléoside est représenté par la formule

$$-(CH_2)_nNHCOZCO-$$

dans laquelle n est un nombre entier valant 1 à 5, et Z est un radical hydrocarbyle divalent.

8. Procédé selon la revendication 7, dans lequel ledit groupe amide covalent est le groupe

$$-(CH_2)_3NHCO(CH_2)_2CO-.$$

9. Procédé selon l'une quelconque des revendications précédentes, dans lequel ledit nucléoside ou désoxynucléoside, qui est fourni à l'étape (3), a pour formule:

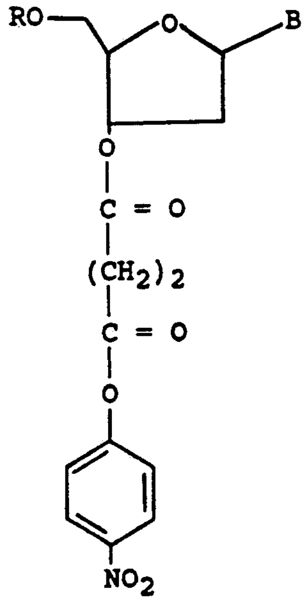

dans laquelle R est un groupe de blocage et B est tel que défini à la revendication 1.